# EUROPEAN PATENT APPLICATION

(11) **EP 2 724 719 A1**
(43) Date of publication of application: **30.04.2014**
(21) Application number: 13190527.5
(22) Date of filing: 05.03.2004
(51) Int. Cl.: A61K 9/50, A61L 24/00, A61L 24/04

(54) **Temporary embolization using inverse thermosensitive polymers**

(30) Priority: 24.03.2003 US 457148 P
(62) Divisional of application: 04718114.4
(71) Applicant: BIOSPHERE MEDICAL, INC., Rockland, MA 02370-1052 (US)
(72) Inventor: Schwarz, Alexander, Brookline, MA Massachusetts 02446 (US); Raymond, Jean, Montreal, Québec H2L 4MI (CA)
(74) Representative: Harrison Goddard Foote LLP

(57) **Abstract**

One aspect of the present invention relates to methods of embolizing a vascular site in a mammal comprising introducing into the vasculature of a mammal a composition comprising an inverse thermosensitive polymer, wherein said inverse thermosensitive polymer gels in said vasculature, which composition may be injected through a small catheter, and which compositions gel at or below body temperature. In certain embodiments of the methods of embolization, said composition further comprises a marker molecule, such as a dye, radiopaque, or an MRI-visible compound.

## Description

### Related Applications

This application claims the benefit of priority to United States Provisional Patent Application serial number 60/457,148, filed March 24, 2003.

### Background of the Invention

### Embolization

In general, an embolization is the therapeutic, temporary or permanent occlusion of a blood vessel. A blood vessel may require occlusion for several reasons including prevention of abnormal bleeding, occlusion of a tumor feeding vessel, or occlusion of an arteriovenous malformation (AVM), which is an abnormal communication between an artery and a vein.

Percutaneous endovascular techniques, such as angioplasty or stenting, usually consist in restoring the patency of diseased vessels. Less frequently, the goal of the intervention is a permanent embolization. During such embolizations, there may also be a need to occlude temporarily normal vessels or branches, to redirect flow-driven particles, or to protect a normal vascular bed from penetration by the embolic agent or from exposure to a cytotoxic drug. In such occasions, it would be beneficial to use an occlusive agent that has a temporary action. This agent should be non-thrombogenic, and the occlusion should be reliably reversible.

The vast majority of the embolization agents used today embolize permanently. However, there are numerous clinical situations, e.g., trauma, postpartum hemorrhage, and GI bleeding, in which temporary embolization is desired. The typical aim of temporary embolization is to block blood flow to the punctured site, allowing the blood vessel to heal over. As a temporary embolization agent degrades, the blood vessel recanalizes, reestablishing the old vasculature.

The temporary embolization agent used most frequently today in the clinical setting is gelfoam. *See generally* Katsumori, T. et al., Am. J. Radiol. 178 (2002) 135 - 139, "Uterine Artery Embolization Using Gelatin Sponge Particles Alone for Symptomatic Uterine Fibroids". This embolic agent comes in the form of sheets. Physicians cut sheet gelfoam into pieces, and inject them into a vessel through a catheter. Gelfoam is degraded by proteases in the blood stream. However, due to differences in enzyme expression from one patient to another, and variation in the size of the pieces of gelfoam used, the in vivo degradation times of this embolization agent span a wide range, i.e., from hours to weeks. Another temporary embolization agent that has been used clinically is starch microspheres. Starch microspheres degrade rapidly, i.e., within minutes to hours, due to the action of α-amylase; unfortunately, this timeframe is too short for most applications.

Balloon angioplasty may also be used for temporary embolization, although it is more frequently used to clear the blocked arteries associated with atherosclerosis. In temporary embolization using balloon angioplasty, a deflated balloon catheter is placed at the arterial site to be embolized; then, the balloon is inflated, thereby blocking blood flow at the site. When the embolization is no longer necessary, the balloon may be deflated and the catheter removed.

Autologous materials, e.g., fat, dura mater, muscle and autologous clot, have also been used for temporary embolization. The main advantage of these materials is their low cost and their inherent biocompatibility. The autologous agent used most frequently is autologous clot. There are several disadvantages associated with using this kind of embolic agent. As noted in connection with gelfoam, the degradation of autologous materials relies on enzymatic action. Because enzyme expression varies from person to person, the degradation time cannot be accurately predicted.

The use of hydrolytically degradable materials for embolization promises to provide a means to exercise control over the *in vivo* lifetime of an embolus. Importantly, enzyme activity would not be a factor in the degradation rate of the embolus. Further, the quantity and pH of the aqueous solution present at the site of embolization can be predicted accurately. Materials comprising hydrolytically degradable polymers have been used to prepare hydrolytically degradable emboli.

Blood vessels, such as arteries, are closed during surgery by clamps and clips. Such devices press against opposite sides of a flexible hollow tube so that the walls flatten out and bear against one another. This produces an axially-extending fold at the two edges. For stopping the flow of fluid through the vessel, this squeezing or pinching action is very effective. However, the lumens of these vessels have linings (intima) which should not be traumatized by strong distortions. Strong pressures, and excessive bending (axial folding), can traumatize them leading to complications after the occluder is removed. Consequently, temporary embolization of blood vessels in the surgical context holds great promise in terms of, for example, patient outcome.

### Poloxamers

Triblock (ABA) copolymers of polyethylene oxide, polypropylene oxide_{b}-polyethylene oxideₐ [PEOₐ-PPO_{b}-PEOₐ], also termed poloxamers (or Pluronics), are nonionic surfactants widely used in diverse industrial applications. Nonionic Surfactants: polyoxyalkylene block copolymers, Vol. 60. Nace VM, Dekker M (editors), New York, 1996. 280 pp. Their surfactant properties have been useful in detergency, dispersion, stabilization, foaming, and emulsification. Cabana A, Abdellatif AK, Juhasz J. Study of the gelation process of polyethylene oxide. polypropylene oxide-polyethylene oxide. copolymer (poloxamer 407) aqueous solutions. Journal of Colloid and Interface Science. 1997;190:307-312. Certain poloxamines, e.g., poloxamine 1307, also display inverse thermosensitivity.

Some of these polymers have been considered for various cardiovascular applications, as well as in sickle cell anemia. Maynard C, Swenson R, Paris JA, Martin JS, Hallstrom AP, Cerqueira MD, Weaver WD.Randomized, controlled trial of RheothRx (poloxamer 188) in patients with suspected acute myocardial infarction. RheothRx in Myocardial Infarction Study Group. Am Heart J. 1998 May; 135(5 Pt 1):797-804; O'Keefe JH, Grines CL, DeWood MA, Schaer GL, Browne K, Magorien RD, Kalbfleisch JM, Fletcher WO Jr, Bateman TM, Gibbons RJ. Poloxamer-188 as an adjunct to primary percutaneous transluminal coronary angioplasty for acute myocardial infarction. Am J Cardiol. 1996 Oct 1;78(7):747-750; and Orringer EP, Casella JF, Ataga KI, Koshy M, Adams-Graves P, Luchtman-Jones L, Wun T, Watanabe M, Shafer F, Kutlar A, Abboud M, Steinberg M, Adler B, Swerdlow P, Terregino C, Saccente S, Files B, Ballas S, Brown R, WojtowiczPraga S, Grindel JM. Purified poloxamer 188 for treatment of acute vasoocclusive crisis of sickle cell disease: A randomized controlled trial. JAMA. 2001 Nov 7;286(17):2099-2106.

Importantly, various members of this class of polymer, e.g., poloxamer 188 and poloxamer 407, show inverse thermosensitivity within the physiological temperature range. Qiu Y, Park K. Environment-sensitive hydrogels for drug delivery. Adv Drug Deliv Rev. 2001 Dec 31;53(3):321-339; and Ron ES, Bromberg LE Temperature-responsive gels and thermogelling polymer matrices for protein and peptide delivery Adv Drug Deliv Rev. 1998 May 4;31(3):197-221. In other words, the two polymers are members of a class that are soluble in aqueous solutions at low temperature, but gel at higher temperatures. Poloxamer 407 is a biocompatible polyoxpropylene-poloxyethylene block copolymer having an average molecular weight of about 12,500 and a polyoxypropylene fraction of about 30%.

Polymers of this type are also referred to as reversibly gelling because their viscosity increases and decreases with an increase and decrease in temperature, respectively. Such reversibly gelling systems are useful wherever it is desirable to handle a material in a fluid state, but performance is preferably in a gelled or more viscous state. As noted above, certain poly(ethyleneoxide)/poly(propyleneoxide) block copolymers have these properties; they are available commercially as Pluronic® poloxamers (BASF, Ludwigshafen, Germany) and generically known as poloxamers. *See* U.S. Pat. Nos. 4,188,373, 4,478,822 and 4,474,751. Further, various poloxamines show inverse thermosensitivity within the physiological temperature range.

### Summary of the Invention

One aspect of the present invention relates to a method of temporarily embolizing a vascular site in a mammal, comprising the step of introducing into the vasculature of a mammal a composition comprising an inverse thermosensitive polymer, wherein said inverse thermosensitive polymer gels in said vasculature, thereby temporarily embolizing a vascular site of said mammal.

In certain embodiments, the present invention relates to the aforementioned method, wherein said mammal is a human.

In certain embodiments, the present invention relates to the aforementioned method, wherein the transition temperature of said inverse thermosensitive polymer is between about 10 C and about 40 C.

In certain embodiments, the present invention relates to the aforementioned method, wherein the volume of the inverse thermosensitive polymer between its transition temperature and physiological temperature is between about 80% and about 150% of the volume of the inverse thermosensitive polymer below its transition temperature.

In certain embodiments, the present invention relates to the aforementioned method, wherein said inverse thermosensitive polymer is a block copolymer, random copolymer, graft polymer, or branched copolymer.

In certain embodiments, the present invention relates to the aforementioned method, wherein said inverse thermosensitive polymer is a block copolymer.

In certain embodiments, the present invention relates to the aforementioned method, wherein said inverse thermosensitive polymer is a polyoxyalkylene block copolymer.

In certain embodiments, the present invention relates to the aforementioned method of temporarily embolizing a vascular site in a mammal, wherein said inverse thermosensitive polymer is a poloxamer or poloxamine.

In certain embodiments, the present invention relates to the aforementioned method of temporarily embolizing a vascular site in a mammal, wherein said inverse thermosensitive polymer is a poloxamer.

In certain embodiments, the present invention relates to the aforementioned method of temporarily embolizing a vascular site in a mammal, wherein said inverse thermosensitive polymer is poloxamer 407, poloxamer 338, poloxamer 188, poloxamine 1107 or poloxamine 1307.

In certain embodiments, the present invention relates to the aforementioned method of temporarily embolizing a vascular site in a mammal, wherein said inverse thermosensitive polymer is poloxamer 407 or poloxamer 338.

In certain embodiments, the present invention relates to the aforementioned method, wherein the transition temperature of said inverse thermosensitive polymer is between about 10 C and about 40 C; and the volume of the inverse thermosensitive polymer between its transition temperature and physiological temperature is between about 80% and about 150% of the volume of the inverse thermosensitive polymer below its transition temperature.

In certain embodiments, the present invention relates to the aforementioned method, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a block copolymer, random copolymer, graft polymer, or branched copolymer.

In certain embodiments, the present invention relates to the aforementioned method of temporarily embolizing a vascular site in a mammal, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a block copolymer.

In certain embodiments, the present invention relates to the aforementioned method, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a polyoxyalkylene block copolymer.

In certain embodiments, the present invention relates to the aforementioned method, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a poloxamer or poloxamine.

In certain embodiments, the present invention relates to the aforementioned method, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a poloxamer.

In certain embodiments, the present invention relates to the aforementioned method, wherein said vascular site is proximal to a surgical incision, hemorrhage, cancerous tissue, uterine fibroid, tumor, or organ.

In certain embodiments, the present invention relates to the aforementioned method, wherein said composition comprising an inverse thermosensitive polymer embolizes said vascular site for less than about twelve hours.

In certain embodiments, the present invention relates to the aforementioned method, wherein said composition comprising an inverse thermosensitive polymer embolizes said vascular site for less than about nine hours.

In certain embodiments, the present invention relates to the aforementioned method, wherein said vascular site is embolized for less than about six hours.

In certain embodiments, the present invention relates to the aforementioned method, wherein said vascular site is embolized for less than about three hours.

In certain embodiments, the present invention relates to the aforementioned method, wherein said vascular site is embolized for less than about two hours.

In certain embodiments, the present invention relates to the aforementioned method, wherein said vascular site is embolized for less than about one hour.

In certain embodiments, the present invention relates to the aforementioned method, wherein said vascular site is embolized for less than about thirty minutes.

In certain embodiments, the present invention relates to the aforementioned method of temporarily embolizing a vascular site in a mammal, wherein the inverse thermosensitive polymer has a polydispersity index from about 1.5 to about 1.0.

In certain embodiments, the present invention relates to the aforementioned method of temporarily embolizing a vascular site in a mammal, wherein the inverse thermosensitive polymer has a polydispersity index from about 1.2 to about 1.0.

In certain embodiments, the present invention relates to the aforementioned method of temporarily embolizing a vascular site in a mammal, wherein the inverse thermosensitive polymer has a polydispersity index from about 1.1 to about 1.0.

In certain embodiments, the present invention relates to the aforementioned method, wherein said composition comprising an inverse thermosensitive polymer further comprises a contrast-enhancing agent.

In certain embodiments, the present invention relates to the aforementioned method, wherein said contrast-enhancing agent is selected from the group consisting of radiopaque materials, paramagnetic materials, heavy atoms, transition metals, lanthanides, actinides, dyes, and radionuclide-containing materials.

In certain embodiments, the present invention relates to the aforementioned method, wherein said composition comprising an inverse thermosensitive polymer further comprises a biologically active agent.

In certain embodiments, the present invention relates to the aforementioned method, wherein the biologically active agent is selected from the group consisting of antiinflammatories, antibiotics, antimicrobials, antivirals, analgesics, antiproliferatives, and chemotherapeutics.

In certain embodiments, the present invention relates to the aforementioned method, wherein said composition comprising an inverse thermosensitive polymer is introduced into the vasculature of said mammal using a catheter.

### Brief Description of the Figures

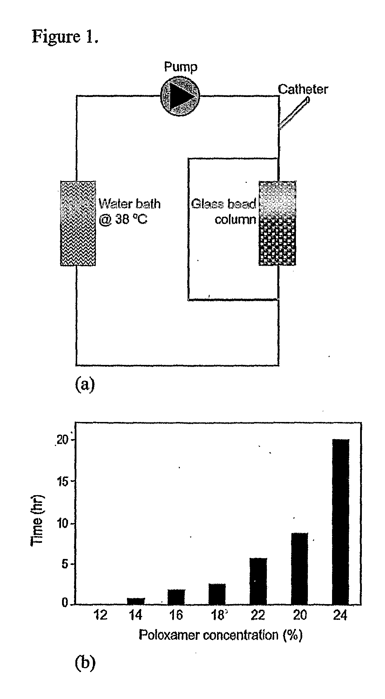

**Figure 1** depicts an *in vitro* model of embolization using a thermosensitive polymer. Figure 1a is a schematic representation of *in vitro* model in which poloxamer 407 is injected through the catheter and gels within the glass bead column, causing redirection of flow around the column until dissolution. Figure 1b is a bar graph illustrating dissolution time as a function of the concentration of poloxamer 407.

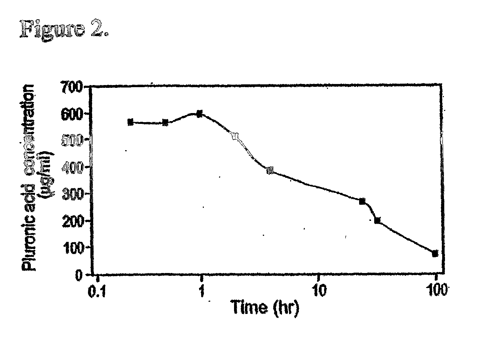

**Figure 2** depicts graphically plasma concentrations of poloxamer 407 at 10 minutes to 120 hours after embolization of the right pulmonary artery in an animal.

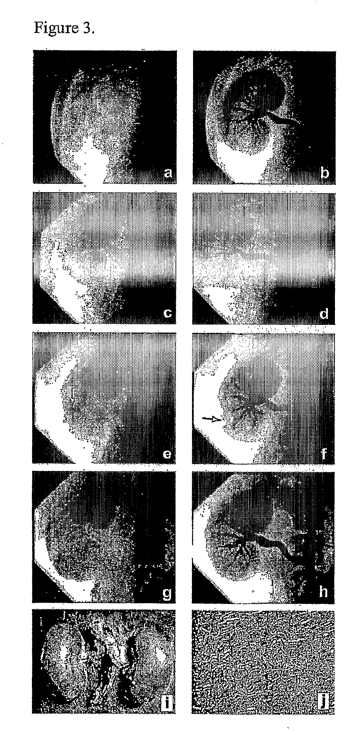

**Figure 3** depicts selected views from renal angiograms (a-h) with (b, d, f, h) or without (a, c, e, g) contrast injection before (a, b), and 5 minutes (c, d), 10 minutes (e, f) and 30 minutes (g, h) after embolization of right renal artery with 3 mL of poloxamer 407 (22%). Note the complete cast of renal branches at 5 minutes (arrows in c, d) with near complete dissolution at 10 minutes (e). A small branch remains occluded at 10 minutes (arrow in e) resulting in a nephrographic defect (arrow in f). The kidney is completely normal at 30 minutes. Macroscopic and pathological studies at one week showed no parenchymal abnormality (i, j) (j: hematoxylin-phloxinesaffron staining; original magnification x50).

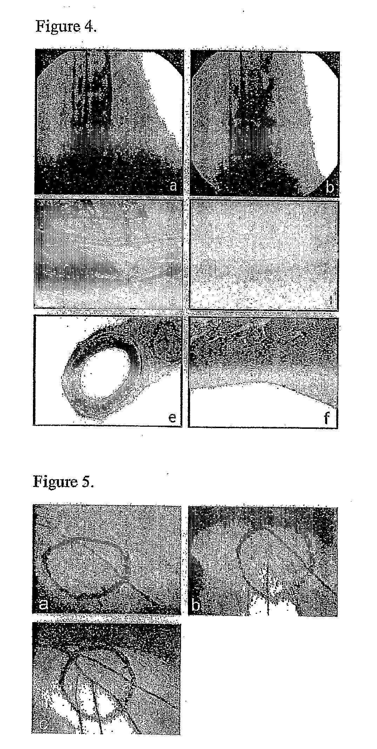

**Figure 4** depicts poloxamer 407 embolization of the left carotid artery. Left carotid arteriogram before embolization (a), and radiograph of poloxamer cast of the left carotid artery (b) immediately before sacrifice. Macroscopic photography immediately after sacrifice (c, d) and pathology (e, f) revealed no vascular injury.
**Figure 5** depicts decanalization of poloxamer 407 occlusions. Macroscopic photography of auricular branches at 10 (a), 60 (b) and 90 minutes (c) after poloxamer embolization of central auricular artery in the rabbit model. Poloxamer 407 is dissolved by blood reaching the cast through collaterals (arrow).

### Detailed Description of the Invention

The invention will now be described more fully with reference to the accompanying examples, in which certain preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

### Overview of a Preferred Embodiment

Remarkably, methods have been developed for safe temporary embolization during intravascular, e.g., catheter-based, and percutaneous endovascular procedures. Poloxamers and poloxamines are non-ionic surfactants with rapid reversible sol-gel transition behavior. The polymers are both safe and efficacious as temporary embolic agents. Initially, dissolution times after gelation of poloxamers and polxamines were determined in an *in vitro* model. Further, for example, transient poloxamer occlusion of renal and pulmonary arteries of seven dogs was followed by serial angiograms. Macroscopic and pathological changes were studied one week later. This experiment was repeated in similar arteries in a pig, and in auricular arteries of two rabbits. Poloxamer dissolution after *in vitro* gelation was completed within 1-20 hours, depending on concentrations. *In vivo* poloxamer 407 (22%) injections led to complete occlusion, followed by full recanalization within 10-90 minutes without complication. The only biochemical effect of poloxamer occlusions was transient elevation of triglyceride levels. There were no pathological abnormalities at one week. For example, poloxamer 407 could be used as a safe and reliable embolic material for temporary occlusions.

### A Preferred Embodiment

Traditional embolization methods for the treatment of vascular diseases rely on blood-flow-directed embolization. In clinical practice, it is sometime desirable to shield a vascular bed from the embolic agent, or to redirect blood flow to the targeted site. Therefore, a short-term and reversible occlusive agent will find use in such procedures.

With respect to temporary embolization, the attraction of inverse thermosensitive polymers is that they can be formulated as a liquid at ambient temperature, which then gels at body temperature. Aqueous solutions of PEO-PPO-PEO block copolymers exhibit interesting temperature-induced aggregation as a result of the hydrophobic nature of the PPO block. For example, at low temperature and concentration, PEO-PPO-PEO block copolymers exist in solution as dissolved monomers, but self-assemble into micelles at higher concentrations and temperatures. Huang K, Lee BP, Ingram DR, Messersmith PB. Synthesis and characterization of self-assembling block copolymers containing bioadhesive end groups Biomacromolecules 2002; 3:397-406. We observed that polymer solutions of poloxamer 407 at a concentration below 12% did not show gelation at any physiological temperature studied, while concentrations above 26% gelled at temperatures that may be too low for practical use.

Remarkably, poloxamer and poloxamine injections led to consistent vascular occlusion at any site, provided the agent could be injected at a sufficient rate to fill the vascular lumen and gel before being carried away by blood flow. Poloxamer and poloxamine occlusions were always transient, with dissolution occurring 5 to 90 minutes after embolization. Poloxamer and poloxamine transient occlusion did not cause any detectable vessel wall damage, either immediately or after one week. Moreover, end organs were unaffected by these short-term occlusions. Further, poloxamer embolization did not affect coagulation times, did not cause thromboembolic complications, and was not associated with vessel spasm.

No ischemic complications occurred after ninety-minute occlusions in a rabbit ear model. The time necessary for dissolution varied according to the completeness of filling and the status of the anatomical vascular bed. No significant difference was observed between arteries and veins, high flow or low flow, high pressure or low pressure, and high resistance or low resistance systems. Probably, dissolution occurs according to the fraction of the total volume of poloxamer in contact with blood. According to this hypothesis, better filling of a vascular bed leads to a longer occlusion time.

Sub-occlusions were rapidly recanalized. In high flow situations, the injection rate had to be high. Injections of polymer that were too slow lead to ineffective embolization, distal embolization with fragmented poloxamer, incomplete occlusions, premature catheter blockage, and rapid dissolution. In that regard, a preferred delivery system comprises a cooled catheter. Such a system prevents catheter blockage and provides better control of poloxamer delivery.

Poloxamers and poloxamines effectively and completely occluded arteries that were then subjected to glue embolization, without affecting cyanoacrylate polymerization. Results of these experiments show that these agents could be used to "protect" a territory during polymer, particulate, or chemo-embolizations.

A potential problem in short-term occlusions is thrombus formation. Poloxamers were found to be antithrombotic and inhibitors of platelet aggregation. Carr ME Jr, Powers PL, Jones MR. Effects of poloxamer 188 on the assembly, structure and dissolution of fibrin clots Thromb Haemost. 1991 Nov 1;66(5):565568; Armstrong JK, Mciselman HJ, Fisher TC. Inhibition of red blood cell-induced platelet aggregation in whole blood by a nonionic surfactant, poloxamer 188 (RheothRx injection) Thromb Res. 1995 Sep 15;79(5-6):437-450; and Carr ME Jr, Carr SL, High AA. Effects of poloxamer 407 on the assembly, structure and dissolution of fibrin clots. Blood Coagul Fibrinolysis 1996 Mar;7(2):109-113. Indeed, in all occlusions performed with poloxamer 407, only one case of thrombus formation was found in a sub-occluded vena cava. The lack of poloxamer thrombogenicity may explain why their use as a femoral closure agent did not succeed: bleeding occurred as soon as femoral arteries recanalized, probably because platelet or fibrin thrombus could not seal the puncture tract.

Poloxamer transient occlusions were associated with transient elevation of triglycerides 24 hours after the procedure. These abnormalities in lipid metabolism have previously been described with systemic infusions of poloxamer. Blonder JM, Baird L, Fulfs JC, Rosenthal GJ. Dose-dependent hyperlipidemia in rabbits following administration of poloxamer 407 gel Life Sci. 1999;65(21):PL261-266.

Poloxamer may also be used as an adjunct tool for devascularization during surgery. The lack of bleeding upon sectioning of arteries could lead to unnoticed vessel trauma and subsequent hemorrages after wound closure. Other potential applications include the use of poloxamers and poloxamines to deliver growth factors or gene therapy. Ron ES, Bromberg LE. Temperature-responsive gels and thermogelling polymer matrices for protein and peptide delivery Adv Drug Deliv Rev. 1998 May 4;31(3):197-221. In sum, poloxamers and poloxamines are safe and effective temporary embolic agents that may be used for protection of vessels during embolization procedures.

### Inverse Thermosensitive Polymers

In general, the inverse thermosensitive polymers used in the methods of the invention, which become a gel at or about body temperature, can be injected into the patient's body in a liquid form. The injected material once reaching body temperature undergoes a transition from a liquid to a gel. The inverse thermosensitive polymers used in connection with the methods of the invention may comprise a block copolymer with reverse thermal gelation properties. The block copolymer can further comprise a polyoxyethylene-polyoxypropylene block copolymer such as a biodegradable, biocompatible copolymer of polyethylene oxide and polypropylene oxide. Also, the inverse thermosensitive polymer can include a therapeutic agent such as an anti-angiogenic agent.

The molecular weight of the inverse thermosensitive polymer is preferably between 1,000 and 50,000, more preferably between 5,000 and 35,000. Preferably the polymer is in an aqueous solution. For example, typical aqueous solutions contain about 1% to about 80% polymer, preferably about 10% to about 40%. The molecular weight of a suitable inverse thermosensitive polymer (such as a poloxamer or poloxamine) may be, for example, between 5,000 and 25,000, and more particularly between 7,000 and 20,000.

The pH of the inverse thermosensitive polymer formulation administered to the mammal is, generally, about 6.0 to about 7.8, which are suitable pH levels for injection into the mammalian body. The pH level may be adjusted by any suitable acid or base, such as hydrochloric acid or sodium hydroxide.

Suitable inverse thermosensitive polymers include polyoxyethylene-polyoxypropylene (PEO-PPO) block copolymers. Two examples are Pluronic® F127 and F108, which are PEO-PPO block copolymers with molecular weights of 12,600 and 14,600, respectively. Each of these compounds is available from BASF of Mount Olive, N.J. Pluronic® F108 at 12-25% concentration in phosphate buffered saline (PBS) is an example of a suitable LCST material. Pluronic® acid F127 at 12-25% concentration in PBS is another example of a suitable material. Low concentrations of dye (such as crystal violet), hormones, therapeutic agents, fillers, and antibiotics can be added to the inverse thermosensitive polymer. For example, a cancer-treating agent, such as endostatin, can be carried by the polymer and thus delivered inside the body along with the inverse thermosensitive polymer. In general, other biocompatible, biodegradable PEO-PPO block copolymers that exist as a gel at body temperature and a liquid at below body temperature may also be used according to the present invention.

Notably, Pluronic® polymers have unique surfactant abilities and extremely low toxicity and immunogenic responses. These products have low acute oral and dermal toxicity and low potential for causing irritation or sensitization, and the general chronic and subchronic toxicity is low. In fact, Pluronic® polymers are among a small number of surfactants that have been approved by the FDA for direct use in medical applications and as food additives (BASF (1990) Pluronic® & Tetronic Surfactants, BASF Co., Mount Olive, N.J.). Recently, several Pluronic® polymers have been found to enhance the therapeutic effect of drugs, and the gene transfer efficiency mediated by adenovirus. (March K L, Madison J E, Trapnell B C. (1995) "Pharmacokinetics of adenoviral vector-mediated gene delivery to vascular smooth muscle cells: modulation by poloxamer 407 and implication for cardiovascular gene therapy." Hum Gene Therapy 6(1): 41-53, 1995).

The average molecular weights of the poloxamers range from about 1,000 to greater than 16,000 daltons. Because the poloxamers are products of a sequential series of reactions, the molecular weights of the individual poloxamer molecules form a statistical distribution about the average molecular weight. In addition, commercially available poloxamers contain substantial amounts of poly(oxyethylene) homopolymer and poly(oxyethylene)/poly(oxypropylene diblock polymers. The relative amounts of these byproducts increase as the molecular weights of the component blocks of the poloxamer increase. Depending upon the manufacturer, these byproducts may constitute from about 15 to about 50% of the total mass of the polymer.

The inverse thermosensitive polymers may be purified using a process for the fractionation of water-soluble polymers, comprising the steps of dissolving a known amount of the polymer in water, adding a soluble extraction salt to the polymer solution, maintaining the solution at a constant optimal temperature for a period of time adequate for two distinct phases to appear, and separating physically the phases. Additionally, the phase containing the polymer fraction of the preferred molecular weight may be diluted to the original volume with water, extraction salt may be added to achieve the original concentration, and the separation process repeated as needed until a polymer having a narrower molecular weight distribution than the starting material and optimal physical characteristics can be recovered.

In certain embodiments, a purified poloxamer or poloxamine has a polydispersity index from about 1.5 to about 1.0. In certain embodiments, a purified poloxamer or poloxamine has a polydispersity index from about 1.2 to about 1.0. In certain embodiments, a purified poloxamer or poloxamine has a polydispersity index from about 1.1 to about 1.0.

The aforementioned process consists of forming an aqueous two-phase system composed of the polymer and an appropriate salt in water. In such a system, a soluble salt can be added to a single phase polymer-water system to induce phase separation to yield a high salt, low polymer bottom phase, and a low salt, high polymer upper phase. Lower molecular weight polymers partition preferentially into the high salt, low polymer phase. Polymers that can be fractionated using this process include polyethers, glycols such as poly(ethylene glycol) and poly(ethylene oxide)s, polyoxyalkylene block copolymers such as poloxamers, poloxamines, and polyoxypropylene/ polyoxybutylene copolymers, and other polyols, such as polyvinyl alcohol. The average molecular weight of these polymers may range from about 800 to greater than 100,000 daltons. *See* U.S. Patent Application 2002/0137973, published September 26, 2002.

The aforementioned purification process inherently exploits the differences in size and polarity, and therefore solubility, among the poloxamer molecules, the poly(oxyethylene) homopolymer and the poly(oxyethylene)/poly(oxypropylene) diblock byproducts. The polar fraction of the poloxamer, which generally includes the lower molecular weight fraction and the byproducts, is removed allowing the higher molecular weight fraction of poloxamer to be recovered. The larger molecular weight poloxamer recovered by this method has physical characteristics substantially different from the starting material or commercially available poloxamer including a higher average molecular weight, lower polydispersity and a higher viscosity in aqueous solution.

Other purification methods may be used to achieve the desired outcome. For example, WO 92/16484 discloses the use of gel permeation chromatography to isolate a fraction of poloxamer 188 that exhibits beneficial biological effects, without causing potentially deleterious side effects. The copolymer thus obtained had a polydispersity index of 1.07 or less, and was substantially saturated. The potentially harmful side effects were shown to be associated with the low molecular weight, unsaturated portion of the polymer, while the medically beneficial effects resided in the uniform higher molecular weight material. Other similarly improved copolymers were obtained by purifying either the polyoxypropylene center block during synthesis of the copolymer, or the copolymer product itself (Emanuele U.S. Pat. No. 5,523,492, Emanuele U.S. Pat. No. 5,696,298).

Further, a supercritical fluid extraction technique has been used to fractionate a polyoxyalkylene block copolymer as disclosed in U.S. Pat. No. 5,567,859. A purified fraction was obtained, which was composed of a fairly uniform polyoxyalkylene block copolymer having a polydispersity of less than 1.17. According to this method, the lower molecular weight fraction was removed in a stream of carbon dioxide maintained at a pressure of 2200 pounds per square inch (psi) and a temperature of 40 C.

Additionally, U.S. Pat. No. 5,800,711 discloses a process for the fractionation of polyoxyalkylene block copolymers by the batchwise removal of low molecular weight species using a salt extraction and liquid phase separation technique. Poloxamer 407 and poloxamer 188 were fractionated by this method. In each case, a copolymer fraction was obtained which had a higher average molecular weight and a lower polydispersity index as compared to the starting material. However, the changes in polydispersity index were modest and analysis by gel permeation chromatography indicated that some low-molecular-weight material remained. The viscosity of aqueous solutions of the fractionated polymers was significantly greater than the viscosity of the commercially available polymers at temperatures between 10 C and 37 C, an important property for some medical and drug delivery applications. Nevertheless, some of the low molecular weight contaminants of these polymers are thought to cause deleterious side effects when used inside the body, making it especially important that they be removed in the fractionation process. As a consequence, polyoxyalkylene block copolymers fractionated by this process are not appropriate for all medical uses.

### Embolization

Embolization is a process wherein a material is injected into a blood vessel to at least partially fill or plug the vessel and/or encourage clot formation so that blood flow through the vessel is reduced or stopped. *See* Background of the Invention. Embolization of a blood vessel can be useful for a variety of medical reasons, including preventing or controlling bleeding due to lesions (e.g., organ bleeding, gastrointestinal bleeding, vascular bleeding, and bleeding associated with an aneurysm), or to ablate diseased tissue (e.g., tumors, vascular malformations, hemorragic processes) by cutting off blood supply. Embolization may also be used to prevent blood loss during or immediately following surgery. Embolization of tumors may be performed preoperatively to shrink tumor size; to aid in the visualization of a tumor; and to minimize or prevent blood loss related to surgical procedures.

In other words, embolization is useful in a broad spectrum of clinical situations. Embolization can be particularly effective in hemorrhage, regardless of whether the etiology is trauma, tumor, epistaxis, postoperative hemorrhage, or GI hemorrhage. It can be performed anywhere in the body that a catheter can be placed, including the intracranial vasculature, head and neck, thorax, abdomen, pelvis, and extremities. With the availability of coaxial microcatheters, highly selective embolizations can be performed. In most patients, embolization for hemorrhage is preferable to surgical alternatives.

Emobilization may be used in treating skin, head, or neck tumors, tumors of the uterus or fallopian tubes, liver or kidney tumors, endometriosis, fibroids, etc. Particularly, embolization has been used for arteriovenous malformation of the pelvis, kidney, liver, spine and brain. Uterine artery embolization has been used for the treatment of fibroids; renal artery embolization has been used for the treatment of renal angiomyolipomas and renal cell carcinoma; intracranial embolization has been used for the treatment of cerebral and intracranial aneurysms, neuroendocrine metastases, intracranial dural arteriovenous fistula and patent ductus arteriosus. Other examples of specific embolization procedures include hepatic artery embolization and pulmonary artery embolization. Examples of such procedures are described, e.g., in Mourikis D., Chatziioannou A., Antoniou A., Kehagias D., Gikas D., Vlahous L., "Selective Arterial Embolization in the Management of Symptomatic Renal Angiomyolipomas (AMLs)," European Journal of Radiology 32(3):153-9, 1999 Dec.; Kalman D. Varenhorst E., "The Role of Arterial Embolization in Renal Cell Carcinoma," Scandinavian Journal of Urology & Nephrology, 33(3):162-70, 1999 Jun.; Lee W., Kim T S., Chung J W., Han J K., Kim S H., Park J H., "Renal Angiomyolipoma: Embolotherapy with a Mixture of Alcohol and Iodized Oil," Journal of Vascular & Interventional Radiology, 9(2):255-61, 1998 March-April; Layelle I., Flandroy P., Trotteur G., Dondelinger R F., "Arterial Embolization of Bone Metastases: is it Worthwhile?" Journal Belge de Radiologie, 81(5):223-5, 1998 Oct.; Berman, M F., Hartmann A., Mast H., Sciacca R R., Mohr J P., PileSpellman J., Young W L., "Determinants of Resource Utilization in the Treatment of Brain Arteriovenous Malformations," Ajnr: American Journal of Neuroradiology, 20(10):2004-8, 1999 Nov.-Dec.; Shi H B., Suh D C., Lee H K., Lim S M., Kim D H., Choi C G., Lee C S., Rhim S C., "Preoperative Transarterial Embolization of Spinal Tumor: Embolization Techniques and Results," Ajnr: American Journal of Neuroradiology, 20(10):2009-15, 1999 Nov-Dec.; Nagino M., Kamiya J., Kanai M., Uesaka K., Sano T., Yamamoto H., Hayakawa N., Nimura Y., "Right Trisegment Portal Vein Embolization for Biliary Tract Carcinoma: Technique and Clinical Utility," Surgery, 127(2):155-60, 2000 Feb.; Mitsuzaki K., Yamashita Y., Utsunomiva D., Sumi S., Ogata I., Takahashi M., Kawakami S., Ueda S., "Balloon-Occluded Retrograde Transvenous Embolization of a Pelvic Arteriovenous Malformation," Cardiovascular & Interventional Radiology 22(6):518-20, 1999 Nov-Dec.

In many instances, embolization procedures begin with diagnostic angiography to identify the source of bleeding. For example, in epistaxis, angiography of the external carotid artery with attention to the internal maxillary artery can be helpful. In pelvic fractures, the internal iliac arteries are examined angiographically. Selective and superselective angiography is more sensitive in finding the source of bleeding than are nonselective studies. Consequently, clinical suspicion and the results of other imaging studies, such as contrast-enhanced CT and radionuclide scans with Technetium Tc 99m-labeled RBCs, are important in guiding angiographic examination. In intra-abdominal bleeding, such as after complex trauma, CT scan may identify the site of acute bleeding, because acute bleeding often demonstrates higher density (Hounsfield units) than older blood; this is termed the "sentinel clot sign."

An embolizing agent, e.g., a thermosensitive polymer, is usually delivered using a catheter. The catheter delivering the embolizing agent composition may be a small diameter medical catheter. The particular catheter employed is not critical, provided that the catheter components and the embolizing agent are mutually compatible. In this regard, polyethylene catheter components can be useful. Other materials compatible with the embolizing agent composition may include fluoropolymers and silicone.

Once a catheter is in place, an embolizing agent composition is injected through the catheter slowly, typically with the assistance of X-ray or fluoroscopic guidance. The embolizing agent composition may be introduced directly into critical blood vessels or they may be introduced upstream of target vessels. The amount of embolizing agent composition introduced during an embolization procedure will be an amount sufficient to cause embolization, e.g., to reduce or stop blood flow through the target vessels. The amount of embolizing agent composition delivered can vary depending on, e.g., the total size or area of the vasculature to be embolized. Adjustment of such factors is within the skill of the ordinary artisan in the embolizing art. After embolization, another arteriogram may be performed to confirm the completion of the procedure. Arterial flow will still be present to some extent to healthy body tissue proximal to the embolization, while flow to the diseased or targeted tissue is blocked.

The embolizing agent composition can preferably comprise a contrast-enhancing agent, which can be tracked and monitored by known methods, including radiography and fluoroscopy. The contrast-enhancing agent can be any material capable of enhancing contrast in a desired imaging modality (e.g., magnetic resonance, X-ray (e.g., CT), ultrasound, magnetotomography, electrical impedance imaging, light imaging (e.g. confocal microscopy and fluorescence imaging) and nuclear imaging (e.g. scintigraphy, SPECT and PET)). Contrast-enhancing agents are well known in the arts of embolization and similar medical practices, with any of a variety of such contrast-enhancing agents being suitable for use in the formulation and methods of the invention.

Certain preferred embodiments include a contrast-enhancing agent that is radiopaque; in particular, a radiopaque material which exhibits permanent radiopacity, e.g., a metal or metal oxide. Permanent radiopacity is unlike some other contrast-enhancing agents or radiopaque materials used in embolization or similar medical applications which biodegrade or otherwise lose their effectiveness (radiopacity) over a certain period, e.g., days or weeks, such as 7 to 14 days. (See, e.g., PCT/GB98/02621). Permanent radiopaque materials are often preferable because they can be monitored or tracked for as long as they remain in the body, whereas other non-permanent contrast-enhancing agents or radiopaque materials have a limited time during which they may be detected and tracked.

Radiopaque materials include paramagnetic materials (e.g., persistent free radicals or more preferably compounds, salts, and complexes of paramagnetic metal species, for example transition metal or lanthanide ions); heavy atom (i.e., atomic number of 37 or more) compounds, salts, or complexes (e.g., heavy metal compounds, iodinated compounds, etc.); radionuclide-containing compounds, salts, or complexes (e.g., salts, compounds or complexes of radioactive metal isotopes or radiodinated organic compounds); and superparamagentic particles (e.g., metal oxide or mixed oxide particles, particularly iron oxides). Preferred paramagnetic metals include Gd (III), Dy (III), Fe (II), Fe (III), Mn (III) and Ho (III), and paramagnetic Ni, Co and Eu species. Preferred heavy metals include Pb, Ba, Ag, Au, W, Cu, Bi and lanthanides, such as Gd.

The amount of contrast-enhancing agent used should be sufficient to allow detection of the embolus as desired. Preferably, the embolizing agent composition can comprise from about 1 to about 50 weight percent of contrast-enhancing agent. The difference in concentration for radiopaque material is as follows: For example, in preferred embodiments, the inverse thermosensitive polymer mixture contains about 50 vol% radiopaque contrast agent solution, wherein preferred contrast agents, e.g., Omnipaque or Visipaque, are non-ionic. For MRI detection, the concentration of the MR detection agent is preferably about 1 weight%.

### Selected Clinical Application of Embolization

As discussed above, embolization typically is performed using angiographic techniques with guidance and monitoring, e.g., fluoroscopic or X-ray guidance, to deliver an embolizing agent to vessels or arteries. Further, a vasodilator (e.g., adenosine) may be administered to the patient beforehand, simultaneously, or subsequently, to facilitate the procedure.

Importantly, while portions of the subsequent description include language relating to specific clinical applications of embolization, all types of embolization processes are considered to be within the contemplation of the methods of the present invention. Specifically, one of skill in the medical or embolizing art will understand and appreciate how microparticles of hydrolytically degradable hydro gels as described herein can be used in various embolization processes by guiding a delivery mechanism to a desired vascular body site, and delivering an amount of the microparticles to the site, to cause restriction, occlusion, filling, or plugging of one or more desired vessels and reduction or stoppage of blood flow through the vessels. Factors that might be considered, controlled, or adjusted for, in applying the process to any particular embolization process might include the chosen composition of the microparticles (e.g., to account for imaging, tracking, and detection of a radiopaque particle substrate); the amount of microparticles delivered to the body site; the method of delivery, including the particular equipment (e.g., catheter) used and the method and route used to place the dispensing end of the catheter at the desired body site, etc. Each of these factors will be appreciated by one of ordinary skill, and can be readily dealt with to apply the described methods to innumerable embolization processes.

### A. Head and Neck

In the head and neck, embolotherapy most often is performed for epistaxis and traumatic hemorrhage. Otorhinolaryngologists differentiate anterior and posterior epistaxis on anatomic and clinical bases. Epistaxis results from a number of causes, including environmental factors such as temperature and humidity, infection, allergies, trauma, tumors, and chemical irritants. An advantage of embolization over surgical ligation is the more selective blockade of smaller branches. By embolizing just the bleeding branch, normal blood flow to the remainder of the internal maxillary distribution is retained. Complications of embolization may include the reflux of embolization material outside the intended area of embolization, which, in the worst case, may result in stroke or blindness. Embolization has been proven more effective than arterial ligation. Although embolization has a higher rate of minor complications, no difference in the rate of major complications was found. For traumatic hemorrhage, the technique of embolization is the same as for epistaxis. Because of the size of the arteries in the head and neck, microcatheters are often required.

### B. Thorax

In the thorax, the two main indications for embolization in relation to hemorrhage are: (1) pulmonary arteriovenous malformations (PAVM); and (2) hemoptysis. PAVMs usually are congenital lesions, although they may occur after surgery or trauma. The congenital form is typically associated with hereditary hemorrhagic telangiectasia, also termed Rendu-Osler-Weber syndrome. There is a genetic predisposition to this condition. PAVMs can be single or multiple, and if large enough, can result in a physiologic right-to-left cardiac shunt. Clinical manifestations of the shunt include cyanosis and polycythemia. Stroke and brain abscesses can result from paradoxical embolism. PAVMs also may hemorrhage, which results in hemoptysis.

Treatment options for PAVMs include surgery and transcatheter therapy. The treatment objective is to relieve the symptoms of dyspnea and fatigue associated with the right-to-left shunt. In addition, if the patient suffers from paradoxical embolism, treatment prevents further episodes. As a result of the less invasive nature of the procedure and excellent technical success rate, embolization currently is considered the treatment of choice for PAVM, whether single or multiple. Embolotherapy is the clear treatment of choice for PAVMs.

Bronchial artery embolization is performed in patients with massive hemoptysis, defined as 500 cm³ of hemoptysis within a 24-hour period. Etiologies vary and include bronchiectasis, cystic fibrosis, neoplasm, sarcoidosis, tuberculosis, and other infections. Untreated, massive hemoptysis carries a high mortality rate. Death most often results from asphyxiation rather than exsanguination. Medical and surgical treatments for massive hemoptysis usually are ineffective, with mortality rates ranging from 35-100%. Embolization has an initial success rate of 95%, with less morbidity and mortality than surgical resection. Consequently, transcatheter embolization has become the therapy of choice for massive hemoptysis, with surgical resection currently reserved for failed embolization or for recurrent massive hemoptysis following multiple prior embolizations.

### C. Abdomen and Pelvis

Many indications for embolization in the abdomen and pelvis exist. For embolization of hemorrhage, the most common indication is acute GI hemorrhage. Solid organ injury, usually to the liver and spleen, can readily be treated with embolization. Other indications exist, such as gynecologic/obstetric-related hemorrhage and pelvic ring fractures.

Once the source of bleeding is identified, an appropriate embolization procedure can be planned. The technique for embolization is different for upper GI bleeding and lower GI bleeding. The vascular supply in the UGI tract is so richly collateralized that relatively nonselective embolizations can be performed without risk of infarcting the underlying organs. Conversely, the LGI tract has less collateral supply, which necessitates more selective embolizations.

Outside the GI tract, there are organ specific considerations when performing embolizations in the abdomen. For instance, the liver has a dual blood supply, with 75% of the total supply from the portal vein and 25% from the hepatic artery. The hepatic artery invariably is responsible for hemorrhage resulting from trauma due to its higher blood pressure compared to the portal vein. Therefore, all embolizations in the liver are performed in the hepatic artery and not in the portal vein. Because of the dual blood supply, occlusion of large branches of the hepatic artery can be performed without risk of necrosis.

In contrast, embolizations of the spleen always should be performed as distally as possible. Occlusion of the splenic artery can result in splenic necrosis and the possibility of a splenic abscess postembolization. If occlusion of the entire splenic artery is contemplated for traumatic hemorrhage, total splenectomy instead of embolization or total splenectomy postembolization should be performed.

Further indications for hemorrhage embolization in the abdomen and pelvis include postpartum, postcesarean, and postoperative bleeding. Differential diagnoses for postpartum bleeding include laceration of the vaginal wall, abnormal placentation, retained products of conception, and uterine rupture. Conservative measures for treating postpartum bleeding include vaginal packing, dilatation and curettage to remove retained products, IV and intramuscular medications (e.g., oxytocin, prostaglandins), and uterine massage. When conservative methods fail, embolization is a safe and effective procedure for controlling pelvic hemorrhage, avoids surgical risks, preserves fertility, and shortens hospital stays.

Finally, embolization of the internal iliac arteries is valuable in patients with hemodynamically unstable pelvic fractures. Protocols for trauma include treatment of associated soft-tissue injury first, followed by stabilization of the pelvic ring. Patients with persistent hemodynamic instability are candidates for embolization. As in other clinical settings, angiography is used to identify the source of hemorrhage, and a selective embolization is performed.

### Embolization in Conjunction with Drug Delivery

New ways of delivering drugs at the right time, in a controlled manner, with minimal side effects, and greater efficacy per dose are sought by the drug delivery and pharmaceutical industries. The reversibly gelling polymers used in the embolization methods of the invention have physico-chemical characteristics that make them suitable delivery vehicles for conventional small-molecule drugs, as well as new macromolecular (e.g., peptides) drugs or other therapeutic products. Therefore, the compositon comprising the thermosensitive polymer may further comprise a pharmaceutic agent selected to provide a pre-selected pharmaceutic effect. A pharmaceutic effect is one which seeks to treat the source or symptom of a disease or physical disorder. Pharmaceutics include those products subject to regulation under the FDA pharmaceutic guidelines, as well as consumer products. Importantly, the compositions used embolization methods of the invention are capable of solubilizing and releasing bioactive materials. Solubilization is expected to occur as a result of dissolution in the bulk aqueous phase or by incorporation of the solute in micelles created by the hydrophobic domains of the poloxamer. Release of the drug would occur through diffusion or network erosion mechanisms.

Those skilled in the art will appreciate that the compositions used in the embolization methods of the invention may simultaneously be utilized to deliver a wide variety of pharmaceutic and personal care applications. To prepare a pharmaceutic composition, an effective amount of pharmaceutically active agent(s) which imparts the desirable pharmaceutic effect is incorporated into the reversibly gelling composition used in the embolization methods of the invention. Preferably, the selected agent is water soluble, which will readily lend itself to a homogeneous dispersion throughout the reversibly gelling composition. It is also preferred that the agent(s) is nonreactive with the composition. For materials which are not water soluble, it is also within the scope of the embolization methods of the invention to disperse or suspend lipophilic material throughout the composition. Myriad bioactive materials may be delivered using the methods of the present invention; the delivered bioactive material includes anesthetics, antimicrobial agents (antibacterial, antifungal, antiviral), anti-inflammatory agents, diagnostic agents, and wound healing agents.

Because the reversibly gelling composition used in the methods of the present invention are suited for application under a variety of physiological conditions, a wide variety of pharmaceutically active agents may be incorporated into and administered from the composition. The pharmaceutic agent loaded into the polymer networks of the thermosensitive polymer may be any substance having biological activity, including proteins, polypeptides, polynucleotides, nucleoproteins, polysaccharides, glycoproteins, lipoproteins, and synthetic and biologically engineered analogs thereof.

A vast number of therapeutic agents may be incorporated in the polymers used in the methods of the present invention. In general, therapeutic agents which may be administered via the methods of the invention include, without limitation: antiinfectives such as antibiotics and antiviral agents; analgesics and analgesic combinations; anorexics; antihelmintics; antiarthritics; antiasthmatic agents; anticonvulsants; antidepressants; antidiuretic agents; antidiarrheals; antihistamines; antiinflammatory agents; antimigraine preparations; antinauseants; antineoplastics; antiparkinsonism drugs; antipruritics; antipsychotics; antipyretics, antispasmodics; anticholinergics; sympathomimetics; xanthine derivatives; cardiovascular preparations including calcium channel blockers and beta-blockers such as pindolol and antiarrhythmics; antihypertensives; diuretics; vasodilators including general coronary, peripheral and cerebral; central nervous system stimulants; cough and cold preparations, including decongestants; hormones such as estradiol and other steroids, including corticosteroids; hypnotics; immunosuppressives; muscle relaxants; parasympatholytics; psychostimulants; sedatives; and tranquilizers; and naturally derived or genetically engineered proteins, polysaccharides, glycoproteins, or lipoproteins. Suitable pharmaceuticals for parenteral administration are well known as is exemplified by the Handbook on Injectable Drugs, 6th edition, by Lawrence A. Trissel, American Society of Hospital Pharmacists, Bethesda, Md., 1990 (hereby incorporated by reference).

The pharmaceutically active compound may be any substance having biological activity, including proteins, polypeptides, polynucleotides, nucleoproteins, polysaccharides, glycoproteins, lipoproteins, and synthetic and biologically engineered analogs thereof. The term "protein" is art-recognized and for purposes of this invention also encompasses peptides. The proteins or peptides may be any biologically active protein or peptide, naturally occurring or synthetic.

Examples of proteins include antibodies, enzymes, growth hormone and growth hormone-releasing hormone, gonadotropin-releasing hormone, and its agonist and antagonist analogues, somatostatin and its analogues, gonadotropins such as luteinizing hormone and follicle-stimulating hormone, peptide T, thyrocalcitonin, parathyroid hormone, glucagon, vasopressin, oxytocin, angiotensin I and II, bradykinin, kallidin, adrenocorticotropic hormone, thyroid stimulating hormone, insulin, glucagon and the numerous analogues and congeners of the foregoing molecules. The pharmaceutical agents may be selected from insulin, antigens selected from the group consisting of MMR (mumps, measles and rubella) vaccine, typhoid vaccine, hepatitis A vaccine, hepatitis B vaccine, herpes simplex virus, bacterial toxoids, cholera toxin B-subunit, influenza vaccine virus, bordetela pertussis virus, vaccinia virus, adenovirus, canary pox, polio vaccine virus, plasmodium falciparum, bacillus calmette geurin (BCG), klebsiella pneumoniae, HIV envelop glycoproteins and cytokins and other agents selected from the group consisting of bovine somatropine (sometimes referred to as BST), estrogens, androgens, insulin growth factors (sometimes referred to as IGF), interleukin I, interleukin II and cytokins. Three such cytokins are interferon-.beta., interferon-.gamma, and tuftsin.

Examples of bacterial toxoids that may be incorporated in the compositions used in the embolization methods of the invention are tetanus, diphtheria, pseudomonas A, mycobaeterium tuberculosis. Examples of that may be incorporated in the compositions used in the embolization methods of the invention are HIV envelope glycoproteins, e.g., gp 120 or gp 160, for AIDS vaccines. Examples of anti-ulcer H2 receptor antagonists that may be included are ranitidine, cimetidine and famotidine, and other anti-ulcer drugs are omparazide, cesupride and misoprostol. An example of a hypoglycaemic agent is glizipide.

Classes of pharmaceutically active compounds which can be loaded into that may be incorporated in the compositions used in the embolization methods of the invention include, but are not limited to, anti-AIDS substances, anti-cancer substances, antibiotics, immunosuppressants (e.g., cyclosporine) anti-viral substances, enzyme inhibitors, neurotoxins, opioids, hypnotics, antihistamines, lubricants tranquilizers, anti-convulsants, muscle relaxants and anti-Parkinson substances, anti-spasmodics and muscle contractants, miotics and anti-cholinergics, anti-glaucoma compounds, anti-parasite and/or anti-protozoal compounds, anti-hypertensives, analgesics, anti-pyretics and anti-inflammatory agents such as NSAIDs, local anesthetics, ophthalmics, prostaglandins, anti-depressants, anti-psychotic substances, anti-emetics, imaging agents, specific targeting agents, neurotransmitters, proteins, cell response modifiers, and vaccines.

Exemplary pharmaceutical agents considered to be particularly suitable for incorporation in the compositions used in the embolization methods of the invention include but are not limited to imidizoles, such as miconazole, econazole, terconazole, saperconazole, itraconazole, metronidazole, fluconazole, ketoconazole, and clotrimazole, luteinizing-hormone-releasing hormone (LHRH) and its analogues, nonoxynol-9, a GnRH agonist or antagonist, natural or synthetic progestrin, such as selected progesterone, 17-hydroxyprogeterone derivatives such as medroxyprogesterone acetate, and 19-nortestosterone analogues such as norethindrone, natural or synthetic estrogens, conjugated estrogens, estradiol, estropipate, and ethinyl estradiol, bisphosphonates including etidronate, alendronate, tiludronate, resedronate, clodronate, and pamidronate, calcitonin, parathyroid hormones, carbonic anhydrase inhibitor such as felbamate and dorzolamide, a mast cell stabilizer such as xesterbergsterol-A, lodoxamine, and cromolyn, a prostaglandin inhibitor such as diclofenac and ketorolac, a steroid such as prednisolone, dexamethasone, fluromethylone, rimexolone, and lotepednol, an antihistamine such as antazoline, pheniramine, and histiminase, pilocarpine nitrate, a beta-blocker such as levobunolol and timolol maleate. As will be understood by those skilled in the art, two or more pharmaceutical agents may be combined for specific effects. The necessary amounts of active ingredient can be determined by simple experimentation.

By way of example only, any of a number of antibiotics and antimicrobials may be included in the thermosensitive polymers used in the methods of the invention. Antimicrobial drugs preferred for inclusion in compositions used in the embolization methods of the invention include salts of lactam drugs, quinolone drugs, ciprofloxacin, norfloxacin, tetracycline, erythromycin, amikacin, triclosan, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole and amanfadine and the like.

By way of example only, in the case of anti-inflammation, non-steroidal anti-inflammatory agents (NSAIDS) may be incorporated in the compositions used in the embolization methods of the invention, such as propionic acid derivatives, acetic acid, fenamic acid derivatives, biphenylcarboxylic acid derivatives, oxicams, including but not limited to aspirin, acetaminophen, ibuprofen, naproxen, benoxaprofen, flurbiprofen, fenbufen, ketoprofen, indoprofen, pirprofen, carporfen, and bucloxic acid and the like.

### Embolization Kits

The methods of the present invention may also be practiced using an embolization kit comprising, for example, poloxamer 407. Such kits may contain a thermosensitive polymer in sterile form, and may include a sterile container of an acceptable reconstitution liquid. Suitable reconstitution liquids are disclosed in Remington's Pharmaceutical Sciences and The United States Pharmacopia--The National Formulary. Such kits may alternatively contain a sterile container of a composition of, for example, poloxamer 407. Such kits may also include, if desired, other conventional kit components, such as, for example, one or more carriers, one or more additional vials for mixing. Instructions, either as inserts or labels, indicating quantities of the embolic composition and carrier, guidelines for mixing these components, and protocols for administration may also be included in the kit. Sterilization of the containers and any materials included in the kit and lyophilization (also referred to as freeze-drying) of the embolic composition may be carried out using conventional sterilization and lyophilization methodologies known to those skilled in the art.

Lyophilization aids useful in the embolization kits include but are not limited to mannitol, lactose, sorbitol, dextran, Ficoll, and polyvinylpyrrolidine(PVP). Stabilization aids useful in the embolization kits include but are not limited to ascorbic acid, cysteine, monothioglycerol, sodium bisulfite, sodium metabisulfite, gentisic acid, and inositol. Bacteriostats useful in the embolization kits include but are not limited to benzyl alcohol, benzalkonium chloride, chlorobutanol, and methyl, propyl or butyl paraben. A component in an embolization kit can also serve more than one function. A reducing agent can also serve as a stabilization aid, a buffer can also serve as a transfer ligand, a lyophilization aid can also serve as a transfer, ancillary or co-ligand and so forth.

The absolute and relative amounts of each component of an embolization kit are determined by a variety of considerations that are in some cases specific for that component and in other cases dependent on the amount of another component or the presence and amount of an optional component. In general, the minimal amount of each component is used that will give the desired effect of the formulation. The desired effect of the formulation is that the end-user of the embolization kit may practice the embolization methods of the invention with a high degree of certainty that the subject will not be harmed.

The embolization kits also contain written instructions for the practicing end-user. These instructions may be affixed to one or more of the vials or to the container in which the vial or vials are packaged for shipping or may be a separate insert, termed the package insert.

### Definitions

For convenience, certain terms employed in the specification, examples, and appended claims are collected here.

The articles "a" and "an" are used herein to refer to one or to more than one (i.e. to at least one) of the grammatical obj ect of the article. By way of example, "an element" means one element or more than one element.

The terms "reversibly gelling" and "inverse thermosensitive" refer to the property of a polymer wherein gelation takes place upon an increase in temperature, rather than a decrease in temperature.

The term "transition temperature" refers to the temperature or temperature range at which gelation of an inverse thermosensitive polymer occurs.

The term "contrast-enhancing" refers to materials capable of being monitored during injection into a mammalian subject by methods for monitoring and detecting such materials, for example by radiography or fluoroscopy. An example of a contrast-enhancing agent is a radiopaque material. Contrast-enhancing agents including radiopaque materials may be either water soluble or water insoluble. Examples of water soluble radiopaque materials include metrizamide, iopamidol, iothalamate sodium, iodomide sodium, and meglumine. Examples of water insoluble radiopaque materials include metals and metal oxides such as gold, titanium, silver, stainless steel, oxides thereof, aluminum oxide, zirconium oxide, etc.

As used herein, the term "polymer" means a molecule, formed by the chemical union of two or more oligomer units. The chemical units are normally linked together by covalent linkages. The two or more combining units in a polymer can be all the same, in which case the polymer is referred to as a homopolymer. They can be also be different and, thus, the polymer will be a combination of the different units. These polymers are referred to as copolymers.

The term "biocompatible", as used herein, refers to having the property of being biologically compatible by not producing a toxic, injurious, or immunological response in living tissue.

The term "degradable", as used herein, refers to having the property of breaking down or degrading under certain conditions, e.g., at neutral or basic pH.

The term "biodegradable", as used herein, refers to a material that undergoes decomposition when contacted with a biological system, such as upon introduction into an animal. The decomposition can be evidenced, for example, by dissolution, depolymerization, disintegration, or by another chemical or physical change, whereby the bulk of the material in the biological system is reduced over time. The decomposition may be, but is not necessarily, catalyzed by a component of the biological system (e.g., an enzyme).

The term "poloxamer" denotes a symmetrical block copolymer, consisting of a core of PPG polyoxyethylated to both its terminal hydroxyl groups, i.e. conforming to the interchangable generic formula (PEG)_{X}-(PPG)_{Y}-(PEG)_{X} and (PEO)_{X}-(PPO)_{Y}-(PEO)_{X}. Each poloxamer name ends with an arbitrary code number, which is related to the average numerical values of the respective monomer units denoted by X and Y.

The term "poloxamine" denotes a polyalkoxylated symmetrical block copolymer of ethylene diamine conforming to the general type [(PEG)_{X}-(PPG)_{Y}]₂-NCH₂CH₂N-[(PPG)_{Y}-(PEG)_{X}]₂. Each Poloxamine name is followed by an arbitrary code number, which is related to the average numerical values of the respective monomer units denoted by X and Y.

The term "inverse thermosensitive polymer" as used herein refers to a polymer that is soluble in water at ambient temperature, but at least partially phase-separates out of water at physiological temperature. Inverse thermosensitive polymers include poloxamer 407, poloxamer 188, Pluronic® F127, Pluronic® F68, poly(N-isopropylacrylamide), poly(methyl vinyl ether), poly(N-vinylcaprolactam); and certain poly(organophosphazenes). *See* Bull. Korean Chem. Soc. 2002, 23, 549-554.

The phrase "polydispersity index" refers to the ratio of the "weight average molecular weight" to the "number average molecular weight" for a particular polymer; it reflects the distribution of individual molecular weights in a polymer sample.

The phrase "weight average molecular weight" refers to a particular measure of the molecular weight of a polymer. The weight average molecular weight is calculated as follows: determine the molecular weight of a number of polymer molecules; add the squares of these weights; and then divide by the total weight of the molecules.

The phrase "number average molecular weight" refers to a particular measure of the molecular weight of a polymer. The number average molecular weight is the common average of the molecular weights of the individual polymer molecules. It is determined by measuring the molecular weight of *n* polymer molecules, summing the weights, and dividing by *n.*

The term "heteroatom" as used herein means an atom of any element other than carbon or hydrogen. Preferred heteroatoms are boron, nitrogen, oxygen, phosphorus, sulfur and selenium.

The term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. In preferred embodiments, a straight chain or branched chain alkyl has 30 or fewer carbon atoms in its backbone (e.g., C₁-C₃₀ for straight chain, C₃-C₃₀ for branched chain), and more preferably 20 or fewer. Likewise, preferred cycloalkyls have from 3-10 carbon atoms in their ring structure, and more preferably have 5, 6 or 7 carbons in the ring structure.

Unless the number of carbons is otherwise specified, "lower alkyl" as used herein means an alkyl group, as defined above, but having from one to ten carbons, more preferably from one to six carbon atoms in its backbone structure. Likewise, "lower alkenyl" and "lower alkynyl" have similar chain lengths. Preferred alkyl groups are lower alkyls. In preferred embodiments, a substituent designated herein as alkyl is a lower alkyl.

The term "aralkyl", as used herein, refers to an alkyl group substituted with an aryl group (e.g., an aromatic or heteroaromatic group).

The terms "alkenyl" and "alkynyl" refer to unsaturated aliphatic groups analogous in length and possible substitution to the alkyls described above, but that contain at least one double or triple bond respectively.

The terms *ortho*, *meta* and *para* apply to 1,2-, 1,3- and 1,4-disubstituted benzenes, respectively. For example, the names 1,2-dimethylbenzene and *ortho*-dimethylbenzene are synonymous.

The abbreviations Me, Et, Ph, Tf, Nf, Ts, Ms represent methyl, ethyl, phenyl, trifluoromethanesulfonyl, nonafluorobutanesulfonyl, *p*-toluenesulfonyl and methanesulfonyl, respectively. A more comprehensive list of the abbreviations utilized by organic chemists of ordinary skill in the art appears in the first issue of each volume of the *Journal of Organic Chemistry*; this list is typically presented in a table entitled Standard List of Abbreviations. The abbreviations contained in said list, and all abbreviations utilized by organic chemists of ordinary skill in the art are hereby incorporated by reference.

As used herein, the definition of each expression, e.g. alkyl, m, n, etc., when it occurs more than once in any structure, is intended to be independent of its definition elsewhere in the same structure.

The phrase "protecting group" as used herein means temporary substituents which protect a potentially reactive functional group from undesired chemical transformations. Examples of such protecting groups include esters of carboxylic acids, silyl ethers of alcohols, and acetals and ketals of aldehydes and ketones, respectively. The field of protecting group chemistry has been reviewed (Greene, T.W.; Wuts, P.G.M. Protective Groups in Organic Synthesis, 2nd ed.; Wiley: New York, 1991).

For purposes of this invention, the chemical elements are identified in accordance with the Periodic Table of the Elements, CAS version, Handbook of Chemistry and Physics, 67th Ed., 1986-87, inside cover.

### Methods of the Invention

In certain embodiments, the present invention relates to a method of temporarily embolizing a vascular site in a mammal, comprising the step of:
introducing into the vasculature of a mammal a composition comprising an inverse thermosensitive polymer, wherein said inverse thermosensitive polymer gels in said vasculature, thereby temporarily embolizing a vascular site of said mammal.

In certain embodiments, the present invention relates to the aforementioned method of temporarily embolizing a vascular site in a mammal, wherein said mammal is a human.

In certain embodiments, the present invention relates to the aforementioned method of temporarily embolizing a vascular site in a mammal, wherein the transition temperature of said inverse thermosensitive polymer is between about 10 C and about 40 C.

In certain embodiments, the present invention relates to the aforementioned method of temporarily embolizing a vascular site in a mammal, wherein the volume of the inverse thermosensitive polymer between its transition temperature and physiological temperature is between about 80% and about 150% of the volume of the inverse thermosensitive polymer below its transition temperature.

In certain embodiments, the present invention relates to the aforementioned method of temporarily embolizing a vascular site in a mammal, wherein said inverse thermosensitive polymer is a block copolymer, random copolymer, graft polymer, or branched copolymer.

In certain embodiments, the present invention relates to the aforementioned method, wherein said inverse thermosensitive polymer is a block copolymer.

In certain embodiments, the present invention relates to the aforementioned method of temporarily embolizing a vascular site in a mammal, wherein said inverse thermosensitive polymer is a polyoxyalkylene block copolymer.

In certain embodiments, the present invention relates to the aforementioned method of temporarily embolizing a vascular site in a mammal, wherein said inverse thermosensitive polymer is a poloxamer or poloxamine.

In certain embodiments, the present invention relates to the aforementioned method of temporarily embolizing a vascular site in a mammal, wherein said inverse thermosensitive polymer is a poloxamer.

In certain embodiments, the present invention relates to the aforementioned method of temporarily embolizing a vascular site in a mammal, wherein said inverse thermosensitive polymer is poloxamer 407, poloxamer 338, poloxamer 188, poloxamine 1107 or poloxamine 1307.

In certain embodiments, the present invention relates to the aforementioned method of temporarily embolizing a vascular site in a mammal, wherein said inverse thermosensitive polymer is poloxamer 407 or poloxamer 338.

In certain embodiments, the present invention relates to the aforementioned method of temporarily embolizing a vascular site in a mammal, wherein the transition temperature of said inverse thermosensitive polymer is between about 10 C and about 40 C; and the volume of the inverse thermosensitive polymer between its transition temperature and physiological temperature is between about 80% and about 150% of the volume of the inverse thermosensitive polymer below its transition temperature.

In certain embodiments, the present invention relates to the aforementioned method of temporarily embolizing a vascular site in a mammal, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a block copolymer, random copolymer, graft polymer, or branched copolymer.

In certain embodiments, the present invention relates to the aforementioned method of temporarily embolizing a vascular site in a mammal, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a block copolymer.

In certain embodiments, the present invention relates to the aforementioned method of temporarily embolizing a vascular site in a mammal, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a polyoxyalkylene block copolymer.

In certain embodiments, the present invention relates to the aforementioned method of temporarily embolizing a vascular site in a mammal, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a poloxamer or poloxamine.

In certain embodiments, the present invention relates to the aforementioned method of temporarily embolizing a vascular site in a mammal, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a poloxamer.

In certain embodiments, the present invention relates to the aforementioned method of temporarily embolizing a vascular site in a mammal, wherein the inverse thermosensitive polymer has a polydispersity index from about 1.5 to about 1.0.

In certain embodiments, the present invention relates to the aforementioned method of temporarily embolizing a vascular site in a mammal, wherein the inverse thermosensitive polymer has a polydispersity index from about 1.2 to about 1.0.

In certain embodiments, the present invention relates to the aforementioned method of temporarily embolizing a vascular site in a mammal, wherein the inverse thermosensitive polymer has a polydispersity index from about 1.1 to about 1.0.

### Exemplification

The invention now being generally described, it will be more readily understood by reference to the following examples, which are included merely for purposes of illustration of certain aspects and embodiments of the present invention, and are not intended to limit the invention.

### Example 1

### Polymer Formulation

Purified poloxamer 407 (polydispersity index, 1.06) (Hinsbar Laboratories, Clawson, MI, USA) was added slowly to ice-cold saline under stirring at twice the desired concentration for the final formulation. As the poloxamer started to go into solution, ice-cold contrast agent (Omnipaque™ 300, Amersham Health, Princeton, NJ, USA) was added to the final volume. The initial slurry was stirred overnight in an ice bath and then sterilized by filtration. For *in vitro* experiments, a drop of food coloring was added to aid the visual assessment of dissolution.

### Example 2

### In Vitro Model of Temporary Embolization

An in vitro model was used to study the time of dissolution of gels of various concentrations (14-24% (w/w)) of poloxamer 407. The *in vitro* model consisted of a 5 mL column filled with glass beads of 200-400 micron size, mimicking a capillary bed (Fig. 1 a). The column, immersed in a heated water bath at 38°C, was perfused at a flow rate of 400 mL/min using a Harvard pump. A bypass around the column was used for flow diversion around the occlusion. In a typical experiment, 1 mL of the polymer solution was injected via a coaxial catheter 2 centimeters from the top of the glass column. Time to dissolution was determined visually by the disappearance of the gel and reestablishment of flow through the column. Dissolution time of poloxamer 407 according to concentration is illustrated in Fig. 1b. As a rule, dissolution *in vitro* was much delayed as compared to *in vivo* experiments. For example, the 22% (w/w) concentration was found to occlude *in vivo* arteries for 10-90 minutes, while *in vitro* occlusions lasted more than 8 hours.

### Example 3

### Temporary Embolization In Vivo

### In vivo vascular occlusion

Protocols for animal experimentation were approved by the Institutional Animal Care Committee in accordance with guidelines of the Canadian Council on Animal Care. All endovascular procedures were performed under general anesthesia. Eight Beagles weighing 10 to 15 kg were sedated with an intramuscular injection of acepromazine (0.1 mg/kg), glycopyrrolate (0.01 mg/kg), and butorphanol (0.1 mg/kg), and anesthetized with intravenous thiopental (15 mg/kg). Animals were ventilated artificially and maintained under surgical anesthesia with 2% isoflurane. Poloxamer 407 (22%) was kept on ice during interventions. Saline containing syringes were also kept on ice to cool the catheter immediately before poloxamer injections.

Rapid injection through 5-F catheter was then elected for most embolizations (Balt, Montmorency, France). Catheterization was performed by percutaneous transfemoral venous and arterial approaches using 5F introducer sheats (Cordis Corporation, Miami, FL, USA). Animals were subjected to temporary occlusion of the right interlobar pulmonary artery and right renal artery by injection of approximately 3 mL of poloxamer 407 (22%).

All vascular occlusions were serially studied by angiography performed 5, 10, 20 or 30 minutes after embolization and after dissolution of the material. A controlateral renal angiogram was performed in all animals to compare angiographic arterial and parenchymal phases after poloxamer 407 dissolution to the normal kidney. Automated coagulation time was measured before and immediately after each procedure in six dogs using blood drawn from the femoral sheath. Follow-up angiographic studies were repeated at one week to exclude any delayed effects such as neointima formation at the level of the arteries submitted to transient occlusions. Temporary occlusions of various other vascular sites were explored immediately before sacrifice, to avoid clinical complications that could occur even with transient occlusions. These include lumbar and hepatic arteries, circumflex femoral veins, and most frequently the left common carotid artery (n = 5). Occlusion of large veins including iliac veins (n = 3) and cava (n = 3) were also attempted, for venous applications.

Poloxamer 407 occlusions were also tested in two rabbits and a pig, to assess if reliable transient occlusions with poloxamer were specific to the species studied. Embolization of porcine renal, femoral, internal iliac and pulmonary arteries in one animal was performed using the same techniques as described above in dogs. Temporary occlusion of the central auricular artery was also studied in rabbits. Two New Zealand rabbits weighting 2.5-3.0 kg were sedated with an intramuscular injection of acepromazine (0.75 mg/kg) and glycopyrrolate (0.01 mg/kg). Preoperative analgesia was provided with EMLA cream (lidocaine 2.5% and prilocaine 2.5%, AstraZeneca LP). The central artery of the ear was catheterized and embolized with 0.05 and 0.1 mL of poloxamer 407 (22 %) after contrast angiography. The appearance, blood flow and recovery of the artery and status of the ear were assessed and compared to the controlateral ear injected with normal saline only.

All interlobar pulmonary arteries could be occluded, and reliably recanalized within 10 to 20 minutes. The shortest occlusion times were associated with sub-occlusions, the longer times to more complete filling of the vascular lumen from distal to proximal. The renal artery could be completely occluded in all cases. Recanalization occurred at about 80 minutes, often a slightly longer time of occlusion than the one seen at the level of the pulmonary artery. The embolization did not cause any radiographic abnormality and renal angiograms were symmetrical after dissolution (Fig. 3).

Lungs and kidneys were macroscopically intact at autopsy (Fig. 3). The pulmonary or renal arteries did not show histopathological abnormalities. Small focal areas of neointimal thickening were found as frequently on the controlateral side as on the side of poloxamer 407 injections, and were attributed to catheter trauma. The renal and pulmonary parenchymas were normal one week after transient arterial occlusion by poloxamer 407.

The carotid arteries were occluded with poloxamer 407 immediately before sacrifice. The polymer could be found at direct inspection at autopsy. There was no visible change of the lining of the vessel as compared to the controlateral artery (Fig. 4).

High flow large venous structures (n = 3) could be occluded with large amounts of poloxamer 407 injected at a fast rate. Partial occlusion of the cava was accompanied by clot formation in one case, the only visible clot associated with poloxamer 407 use in the entire study. These injections led to the observation that escape of the polymer to the pulmonary bed led to poloxamer emboli that dissolved much more rapidly than direct pulmonary artery injections. Poloxamer 407 embolization in porcine arteries led to the same observations as in the canine model, with approximately 20 minute occlusions at all sites.

### Autopsy

Macroscopic photography of the main arteries and of the end organs was performed at the time of autopsy. Pathological studies were performed on tissue blocks from samples of any visible abnormality, and on random sampling in organs without abnormality. Slides were stained with hematoxylin-phloxin-saffron and Movat's pentachrome stain. Each slide was studied in parallel with a control slide prepared from the artery, vein, or end-organ from the side controlateral to the poloxamer injections.

### Example 4

### Endovascular Temporary Embolization

At the time of the follow-up angiogram, potential endovascular applications were explored. Cyanoacrylate was injected through 2F microcatheters (Target Therapeutics Inc., Boston Scientific Corporation, Fremont, CA, USA) positioned proximal to poloxamer 407 occlusions, to test if the glue could infiltrate the poloxamer, or penetrate between the poloxamer and the vessel wall (n = 4). Complete and permanent arterial occlusions were produced by cyanoacrylate injected proximal to poloxamer 407 in one hepatic artery, one lumbar artery, one circumflex vein, and one carotid artery. Cyanoacrylate could not penetrate beyond the poloxamer gel, nor infiltrate between the poloxamer 407 cast and the vessel wall.

### Example 5

### Temporary Embolization of Femoral Artery Subsequent to Catheter Angiography

Femoral arteries were also temporarily occluded (n = 3) at the time of catheter retrieval to explore the potential of poloxamer 407 as a femoral closure agent after angiography. Catheters could be retrieved from femoral arteries without any compression or bleeding when poloxamer 407 was used for femoral closure. However, after 15 to 32 minutes, the wound suddenly reopened in all cases, necessitating routine compression for hemostasis. The injection of poloxamer 407 did not cause any change in the coagulation time. Results of routine hematology and biochemistry tests are summarized in Table 1.

### Example 6

### Laboratory investigations

Routine hematology and biochemistry multianalyses were performed in four dogs immediately before and after the procedure, at 24 hours and one week. Because many physiological values are disturbed by fasting, anesthesia, angiography, and recovery period, routine laboratory tests were compared to six other dogs submitted to platinum coil embolization. Statistical comparisons were made with Independent-Samples T tests. Poloxamer 407 was used in the poloxamer tests.

**Table 1. Results of routine hematology and biochemistry tests**

| | | **T = 0** | **T = 1 h** | **T = 24 h** | **T = 1 week** |
|---|---|---|---|---|---|
| **Creatinine** | *Poloxamer* | 56.30 | 55.70 | 66.20 | 50.00 |
| | *Control* | 60.00 | 50.00 | 55.00 | 50.00 |
| **Proteins** | *Poloxamer* | 53.00 | 43.30 | 55.20 | 56.00 |
| | *Control* | 47.00 | 39.00 | 52.00 | |
| **Triglycerides** | *Poloxamer* | 0.33 | 0.50 | 1.53* | 0.66 |
| | *Control* | 0.25 | 0.26 | 0.30* | |
| **Cholesterol** | *Poloxamer* | 4.30 | 3.50 | 5.30 | 3.90 |
| | *Control* | 3.50 | 3.00 | 3.60 | |
| **HDL** | *Poloxamer* | 4.00 | 3.20 | 4.10 | 3.30 |
| | *Control* | 3.00 | 3.00 | 3.20 | |
| **LDL** | *Poloxamer* | 0.56 | 0.39 | 0.75 | 0.36 |
| | *Control* | 0.30 | 1.00 | 0.30 | |
| **White Cells** | *Poloxamer* | 6.75 | 6.40 | 13.50 | 5.15 |
| | *Control* | 6.00 | 6.00 | 16.00 | |
| **Platelets** | *Poloxamer* | 290.00 | 271.00 | 294.00 | 215.00 |
| | *Control* | 200.00 | 180.00 | 300.00 | |
| **Hematocrit** | *Poloxamer* | 0.34 | 0.29 | 0.41 | 0.32 |
| | *Control* | 0.33 | 0.28 | 0.42 | |

| | | | | | |
|---|---|---|---|---|---|
| *p=0.031 by an independent-samples T test | | | | | |

In the control systems -- dogs subjected to coil embolization -- there were many similar physiological changes, such as hemodilution immediately after the procedure, hemoconcentration and elevated white blood cell counts at 24 hours, a finding that we attribute to the stress of the procedures. Triglycerides were elevated at 24 hours, an abnormality not found in animals subjected to coil embolization (*See* Table 1 above).

### Example 7

### Temporary Embolization of a Canine Artery -- Rapid Dissolution of an Embolus

The pulmonary artery of a dog was occluded with poloxamer 407. Immediately after, the catheter was exchanged and cold saline was injected proximal to the occlusion. The poloxamer 407 dissolved and the artery was free of any occlusions. This experiment demonstrated the on-demand reversibility of the embolization.

### Example 8

### Temporary Embolization in Rabbits

In two rabbits, the central auricular artery was catheterized and embolized with poloxamer 407 (22 %). Occlusion times were approximately 90 minutes in both animals. Recanalization was directly witnessed by direct observation and magnification (Fig. 5). Dissolution of the material started at the level of arterial segments supplied by collateral branches, in a retrograde fashion. The lumen was recanalized along segmented channels at first. Once this process started, dissolution became accelerated and completed within another 30 minutes. After a period of transient ischemia, the ear appeared normal. Transient spasm, at the tip of the catheter, persisted longer than poloxamer 407 occlusion on both sides. Rabbits were followed for 1 week, without any visible complication at the level of the ear or the central auricular artery.

### Example 9

### Pharmacokinetic study of soluble poloxamer

To determine the half-life of the poloxamer 407 in vivo after dissolution, blood was collected from one dog 15 minutes to 120 hours after occlusion of the right lower lobe pulmonary artery with 3 mL of poloxamer 407 (22%) (w/w). The plasma concentration of poloxamer 407 was determined by HPLC. Briefly, poloxamer 407 was quantitatively recovered from plasma aliquots by repeated extraction with tetrahydrofuran. The extracts were combined and the solvent removed by evaporation under reduced pressure. The residue was redissolved in a known volume of tetrahydrofuran, a derivatization reagent containing an UV absorbing chromophore was added, and the reaction was allowed to proceed to completion. The poloxamer 407 derivative was separated from excess derivatization reagent and plasma components by gel permeation chromatography (GPC-HPLC) and visualized using UV detection. The amount of poloxamer 407 present in the plasma was quantified by comparison of the poloxamer derivative peak area to that of a series of similarly prepared external standards. Limit of detection was approximately 2 µg poloxamer 407 per mL of plasma.

Plasma concentrations of dissoluted poloxamer 407 after transient occlusion of right pulmonary artery with 3 mL are shown in Fig. 2. Poloxamer 407 could not be detected in plasma after 100 hours.

### Example 10

### Embolization using Poloxamer 338

Using the experimental protocol described above, the hepatic artery of a beagle was occluded with approx. 6 mL of cooled, fractionated poloxamer 338 (polydispersity index, 1.08) solution containing 18 wt% polymer and 50% of the radiopaque contrast agent Omnipaque™. The artery stayed occluded for 45 minutes and was reopened by the injection of cold saline. The right pulmonary artery was occluded with about 4 mL of the same poloxamer 338 solution for about 20 minutes, after which the polymer dissolved and the artery was open again.

### Example 11

### Embolization Using Poloxamine 1107

Using the experimental protocol described above, the hepatic artery of a beagle was occluded with approx. 6 mL of cooled, fractionated poloxamine 1107 solution containing 20 wt% polymer and 50% of the radiopaque contrast agent Omnipaque™. The artery stayed occluded for about 20 minutes and reopened by dissolution of the polymer. The right renal artery was occluded with the same poloxamine 1107 solution using about 3 mL and reopened again after about 5 minutes.

### Example 12

### Embolization Using Poloxamine 1307

Using the experimental protocol described above, the hepatic artery of a beagle was embolized with approx. 5 mL of cooled poloxamine 1307 solution containing 21 wt% polymer and 50% of the radiopaque contrast agent Omnipaque™. Blood flow was partially reestablished at 10 minutes and fully reestablished at 15 minutes. Part of the shoulder artery was occluded using the same poloxamine 1307 solution and stayed occluded for about 10 minutes, after which blood flow was reestablished.

### Additional Patents and Publications Cited

1. U.S. Patent Application Publication No. US 2002/0137973 A1.
2. Blood Coagulation and Fibrinolysis, 7, 109-113 (1996).
3. Life Sciences, Vol. 65, No. 21, pp. PL 261-266, (1999).
4. Nonionic Surfactants Polyoxyalkylene Block Copolymers; edited by Vaugh M. Nace; Marcel Dekker, Inc., New York.
5. United States Patent No. 5,834,007
6. International Published Patent Application No. WO 00/45868
7. United States Patent No. 5,525,334
8. J. Biomater. Sci. Polymer Edn. Vol. 7, No. 9, pp. 795-804 (1996).
9. TIBTECH October 2000 (Vol. 18) 412-420.
10. Advanced Drug Delivery Reviews 53 321-339 (2001).
11. Advanced Drug Delivery Reviews 31 197-221 (1998).
12. United States Patent No. 4,268,495.
13. United States Patent No. 6,333,194.
14. United States Patent No. 5,744,163.
15. United States Patent No. 5,605,687.
16. United States Patent No. 5,470,568.
17. U.S. Patent Application Publication No. US 2002/0077461 A1.
18. Schmolka, "A Review of Block Polymer Surfactants" J. Am. Oil Chemists' Soc. 54:110-116 (1977).
19. Matsumaru, Y. et al. "Application of thermosensitive polymers as a new embolic material for intravascular neurosurgery" Journal of Biomaterials Science: Polymer Edition, Vol. 7, No. 9, 795 (1996).
20. United States Patent No. 6,316,011

### Incorporation by Reference

All of the patents and publications cited herein are hereby incorporated by reference.

### Equivalents

Those skilled in the art will recognize, or be able to ascertain using no more than routine experimentation, many equivalents to the specific embodiments of the invention described herein. Such equivalents are intended to be encompassed by the following claims.

The present application and invention further includes the subject matter of the following numbered clauses:
1. A method of temporarily embolizing a vascular site in a mammal, comprising the step of:
   introducing into the vasculature of a mammal a composition comprising an inverse thermosensitive polymer, wherein said inverse thermosensitive polymer gels in said vasculature, thereby temporarily embolizing a vascular site of said mammal.
2. The method of clause 1, wherein said mammal is a human.
3. The method of clause 1, wherein the transition temperature of said inverse thermosensitive polymer is between about 10 C and about 40 C.
4. The method of clause 1, wherein the volume of the inverse thermosensitive polymer between its transition temperature and physiological temperature is between about 80% and about 150% of the volume of the inverse thermosensitive polymer below its transition temperature.
5. The method of clause 1, wherein said inverse thermosensitive polymer is a block copolymer, random copolymer, graft polymer, or branched copolymer.
6. The method of clause 1, wherein said inverse thermosensitive polymer is a block copolymer.
7. The method of clause 1, wherein said inverse thermosensitive polymer is a polyoxyalkylene block copolymer.
8. The method of clause 1, wherein said inverse thermosensitive polymer is a poloxamer or poloxamine.
9. The method of clause 1, wherein said inverse thermosensitive polymer is a poloxamer.
10. The method ofclause 1, wherein said inverse thermosensitive polymer is poloxamer 407, poloxamer 338, poloxamer 188, poloxamine 1107 or poloxamine 1307.
11. The method of clause 1, wherein said inverse thermosensitive polymer is poloxamer 407 or poloxamer 338.
12. The method of clause 1, wherein the transition temperature of said inverse thermosensitive polymer is between about 10 C and about 40 C; and the volume of the inverse thermosensitive polymer between its transition temperature and physiological temperature is between about 80% and about 150% of the volume of the inverse thermosensitive polymer below its transition temperature.
13. The method of clause 1, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a block copolymer, random copolymer, graft polymer, or branched copolymer.
14. The method of clause 1, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a block copolymer.
15. The method of clause 1, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a polyoxyalkylene block copolymer.
16. The method of clause 1, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a poloxamer or poloxamine.
17. The method of clause 1, wherein the transition temperature of said inverse thermosensitive polymer is between about 10 C and about 40 C; the volume of the inverse thermosensitive polymer between its transition temperature and physiological temperature is between about 80% and about 150% of the volume of the inverse thermosensitive polymer below its transition temperature; and said inverse thermosensitive polymer is a poloxamer.
18. The method of clause 1, 2, 8, 9, 10, or 11, wherein said composition comprising an inverse thermosensitive polymer is introduced into the vasculature of said mammal using a catheter.
19. The method of clause 1, 2, 8, 9, 10, or 11, wherein said vascular site is proximal to a surgical incision, hemorrhage, cancerous tissue, uterine fibroid, tumor, or organ.
20. The method of clause 1, 2, 8, 9, 10, or 11, wherein said composition comprising an inverse thermosensitive polymer embolizes said vascular site for less than about twelve hours.
21. The method of clause 1, 2, 8, 9, 10, or 11, wherein said composition comprising an inverse thermosensitive polymer embolizes said vascular site for less than about nine hours.
22. The method of clause 1, 2, 8, 9, 10, or 11, wherein said vascular site is embolized for less than about six hours.
23. The method of clause 1, 2, 8, 9, 10, or 11, wherein said vascular site is embolized for less than about three hours.
24. The method of clause 1, 2, 8, 9, 10, or 11, wherein said vascular site is embolized for less than about two hours.
25. The method of clause 1, 2, 8, 9, 10, or 11, wherein said vascular site is embolized for less than about one hour.
26. The method of clause 1, 2, 8, 9, 10, or 11, wherein said vascular site is embolized for less than about thirty minutes.
27. The method of clause 1, 2, 8, 9,10, or 11, wherein the inverse thermosensitive polymer has a polydispersity index from about 1.5 to about 1.0.
28. The method of clause 1, 2, 8, 9, 10, or 11, wherein the inverse thermosensitive polymer has a polydispersity index from about 1.2 to about 1.0.
29. The method of clause 1, 2, 8, 9,10, or 11, wherein the inverse thermosensitive polymer has a polydispersity index from about 1.1 to about 1.0.
30. The method of clause 1, wherein said composition comprising an inverse thermosensitive polymer further comprises a contrast-enhancing agent.
31. The method of clause 30, wherein said contrast-enhancing agent is selected from the group consisting of radiopaque materials, paramagnetic materials, heavy atoms, transition metals, lanthanides, actinides, dyes, and radionuclide-containing materials.
32. The method of clause 1, wherein said composition comprising an inverse thermosensitive polymer further comprises a biologically active agent.
33. The method of clause 32, wherein the biologically active agent is selected from the group consisting of antiinflammatories, antibiotics, antimicrobials, antivirals, analgesics, antiproliferatives, and chemotherapeutics.

## Claims

1. A composition for temporarily embolizing a vascular site in a mammal for treatment of arteriovenous malformation of the pelvis, kidney, liver, spine and brain and pulmonary arteriovenous malformation comprising:
an aqueous solution comprising 10-40% (w/w) of at least one purified inverse thermosensitive polymer, wherein said at least one purified inverse thermosensitive polymer gels in said vasculature, thereby temporarily embolizing said vascular site of said mammal, wherein said inverse thermosensitive polymer is a poloxamer or poloxamine.

2. The composition of claim 1, wherein the transition temperature of said inverse thermosensitive polymer is between 10°C and 40°C.

3. The composition of claim 1 or claim 2, wherein the volume of the inverse thermosensitive polymer between its transition temperature and physiological temperature is between 80% and 150% of the volume of the inverse thermosensitive polymer below its transition temperature.

4. The composition of any preceding claim, wherein said inverse thermosensitive polymer is poloxamer 407, poloxamer 338, poloxamer 188, poloxamine 1107 or poloxamine 1307.

5. The composition of any preceding claim, wherein said composition comprising an inverse thermosensitive polymer embolizes said vascular site for less than twelve hours.

6. The composition of any preceding claim, wherein said vascular site is embolized for less than nine hours.

7. The composition of any preceding claim, wherein said vascular site is embolized for less than six hours.

8. The composition of any preceding claim, wherein said vascular site is embolized for less than three hours, optionally less than two hours.

9. The composition of any preceding claim, wherein said vascular site is embolized for less than one hour, optionally less than thirty minutes.

10. The composition of any preceding claim, wherein the inverse thermosensitive polymer has a polydispersity index from 1.5 to 1.0.

11. The composition of any preceding claim, wherein the inverse thermosensitive polymer has a polydispersity index from 1.2 to 1.0.

12. The composition of any preceding claim, wherein the inverse thermosensitive polymer has a polydispersity index from 1.1 to 1.0.

13. The composition of any preceding claim, wherein said composition comprising an inverse thermosensitive polymer further comprises a contrast-enhancing agent.

14. The composition of claim 13, wherein said contrast-enhancing agent is selected from the group consisting of radiopaque materials, paramagnetic materials, heavy atoms, transition metals, lanthanides, actinides, dyes, and radionuclide-containing materials.

15. The composition of any preceding claim, wherein the vascular site is proximal to a hemmorrage.
